Europäisches Patentamt

(19) European Patent Office

Office européen des brevets

(11) Veröffentlichungsnummer: **0 158 025**

**A1**

(12) # EUROPÄISCHE PATENTANMELDUNG

(21) Anmeldenummer: **85101231.0**

(22) Anmeldetag: **06.02.85**

(51) Int. Cl.⁴: **A 61 N 5/06**

(30) Priorität **07.02 84 DE 3404214**

(43) Veröffentlichungstag der Anmeldung:
**16.10.85 Patentblatt 85/42**

(84) Benannte Vertragsstaaten:
**AT BE CH DE FR GB IT LI LU NL SE**

(71) Anmelder: **AVARIS AG**
**Seilergraben 49**
**CH-8001 Zürich(CH)**

(72) Erfinder: **Kerschgens, Johann Josef**
**St.-Gotthard-Strasse 44**
**CH-6460 Altdorf(CH)**

(74) Vertreter: **Patentanwälte Dr. Solf & Zapf**
**Asamstrasse 8**
**D-8000 München 90(DE)**

(54) **Vorrichtung zur Hautbestrahlung sowie zur Haartrocknung.**

(57) Die Erfindung betrifft eine Vorrichtung zur Hautbestrahlung sowie Haartrocknung, mit einem Luftführungskanal, in dem ein Gebläse, eine Luftstromheizung und eine ultraviolette Strahlung abgebende Strahlungsquelle angeordnet ist, wobei in einer ersten Betriebsart die eingeschaltete Strahlungsquelle bei der Bestrahlungsbehandlung von einem vom Gebläse erzeugten Luftstrom gekühlt wird, und in einer zweiten Betriebsart ein von dem Gebläse erzeugter und von der Heizung angewärmter, austretender Luftstrom zur Haartrocknung dient. Die erfindung ist dadurch gekennzeichnet, daß sich in dem Luftführungskanal (6) einendig die Strahlungsquelle (15), anderendig die Heizung (16) und zwischen der Strahlungsquelle (15) under der Heizung (16) das Gebläse (17) befindet

FIG. 1

EP 0 158 025 A1

PATENTANWÄLTE

Zugelassene Vertreter beim Europäischen Patentamt
European Patent Attorneys

DR. SOLF 0158025

München - Wuppertal

M 259 EPA

AVARIS AG, Seilergraben 49,
CH-8001  Zürich (Schweiz)

Vorrichtung zur Hautbestrahlung sowie zur
Haartrocknung

Die Erfindung betrifft eine Vorrichtung zur Hautbestrahlung sowie Haartrocknung, mit einem Luftführungskanal, in dem ein Gebläse, eine Luftstromheizung und eine ultraviolette Strahlung abgebende Strahlungsquelle angeordnet ist, wobei in einer ersten Betriebsart die eingeschaltete Strahlungsquelle bei der Bestrahlungsbehandlung von einem vom Gebläse erzeugten Luftstrom gekühlt wird und in einer zweiten Betriebsart ein von dem Gebläse erzeugter und von der Heizung angewärmter, austretender Luftstrom zur Haartrocknung dient.

Ein älterer Vorschlag des Anmelders (deutsche Patentanmeldung P 33 22 071.9-33) betrifft eine Vorrichtung der eingangs genannten Art, mit der sowohl eine Hautbestrahlung als auch eine Haartrocknung vorge-

Asamstraße 8, D-8000 München 90
Telefon (089) 653665; Telefax (089) 653218
Telex: 5214168 soza d

Patentanwalt Dr.-Ing. Dipl.-Ing. A. Solf
Patentanwalt Dipl.-Ing. Chr. Zapf

- 2 -

nommen werden kann. Die mit ultravioletter Strahlung (UV-Strahlung) erfolgende Hautbestrahlung wird durchgeführt, um eine therapeutische und/oder kosmetische Wirkung zu erzielen. Neben der Wirkung der UV-Strahlung zur Bräunung der Haut bzw. zur Erhöhung der Widerstandskraft des Körpers hat sich gezeigt, daß ein auf eine Hauterkrankung (z.B. Schuppenflechte) geleiteter Luftstrom in Verbindung mit einer UV-Strahlung zu einer außerordentlich guten Heilwirkung und schnellen Ausmerzung der Erkrankung führt. In der Betriebsart "Haartrockner" gibt die oben genannte Vorrichtung einen von einer Heizung erwärmten Luftstrom ab. Nach dem älteren Vorschlag des Anmelders ist die Vorrichtung als Haartrockner (Haarfön) ausgeführt, aus dessen Luftaustrittsstutzen ein UV-Strahlungsaufsatz aufgesetzt werden kann, der aus einem Gehäuse besteht, in dem die UV-Strahlungsquelle angeordnet ist. Soll diese Vorrichtung als Haartrockner verwendet werden, so ist der UV-Strahlungsaufsatz von dem Luftaustrittsstutzen abzunehmen; erfolgt der Einsatz der Vorrichtung als Bestrahlungsgerät, so ist der UV-Strahlungsaufsatz wiederum auf den Luftaustrittsstutzen aufzusetzen. Diese Vorrichtung hat sich in der Praxis bewährt, jedoch ist die Handhabung relativ umständlich, da für den Wechsel der Betriebsart jeweils ein Aufstecken bzw. Abnehmen des UV-Strahlungsaufsatzes vorgenommen werden muß. Auch besitzt die Vorrichtung aufgrund der Aufsteckbarkeit des UV-Strahlungsaufsatzes einen relativ komplizierten Aufbau, da Halterungseinrichtungen für den UV-Strahlungsaufsatz vorgesehen sein müssen und eine elektrische Steckverbindungseinrichtung für die Zufuhr der Versorgungsspannung zur UV-Strahlungsquelle notwendig ist.

- 3 -

Der Erfindung liegt daher die Aufgabe zugrunde, eine Vorrichtung der eingangs genannten Art zu schaffen, die bei einem einfachen Aufbau keine Umrüstungsmaßnahmen für einen Wechsel der Betriebsart erfordert.

Diese Aufgabe wird erfindungsgemäß dadurch gelöst, daß sich in dem Luftführungskanal einendig die Strahlungsquelle, anderendig die Heizung und zwischen der Strahlungsquelle und der Heizung das Gebläse befindet. Durch die erfindungsgemäße Ausbildung ist ein Wechsel der Betriebsart durch einfache Betätigung eines Bedien·organs (Schalters) möglich; denn in der ersten Betriebsart ist die Strahlungsquelle ein- und die Heizung ausgeschaltet und das Gebläse fördert einen Luftstrom durch den Luftführungskanal, der die in diesem Luftführungskanal angeordnete Strahlungsquelle kühlt. In der zweiten Betriebsart ist die Heizung ein- und die UV-Strahlungsquelle ausgeschaltet und das Gebläse fördert einen Luftstrom, der sich an der erhitzten Heizung erwärmt und dann zur Haartrocknung aus der Vorrichtung austritt. Hierdurch ist eine sehr einfache Handhabung der erfindungsgemäßen Vorrichtung gewährleistet, denn es entfällt das im Stand der Technik bekannte Aufstecken bzw. Abnehmen eines UV-Strahlungsaufsatzes. Ferner ergibt sich auch ein unkomplizierter Aufbau der erfindungsgemäßen Vorrichtung, da die im Stand der Technik vorhandenen Halterungs- und Steckverbindungseinrichtungen für einen UV-Strahlungsaufsatz entfallen.

Vorteilhafterweise wird man die Richtung des Luftstroms in der zweiten, der Haarbehandlung dienenden Betriebsart

- 3a -

so zu wählen, daß er auf der Seite der Strahlungsquelle in den Luftführungskanal ein- und auf der Seite der Heizung aus dem Luftführungskanal austritt. Um in der ersten, der Bestrahlung dienenden Betriebsart eine Kühlung der Strahlungsquelle vorzunehmen, ist es erforderlich, die Strahlungsquelle in dem Luftstrom des Gebläses anzuordnen. Die Richtung des Luftstroms ist hierbei derart wählbar, daß entweder der Luftstrom auf der Seite der Strahlungsquelle in den Luftführungskanal ein- und auf der Seite der Heizung aus dem Luftführungskanal austritt, so daß die Strahlungsquelle durch die Saugwirkung des Gebläses gekühlt wird, oder andererseits der Luftstrom auf der Seite der Heizung in den Luftführungskanal ein- und auf der Seite der Strahlungsquelle aus dem Luftführungskanal austritt, so daß die Strahlungsquelle durch die Blaswirkung des Gebläses gekühlt wird.

Nach einer Weiterbildung der Erfindung ist vorgesehen, daß das Gebläse mit einem Wechsel der Betriebsart die Richtung des Luftstroms in dem Luftführungskanal umkehrt. Vorteilhafterweise kann dabei vorgesehen sein, daß der Luftstrom in der ersten Betriebsart aus dem Ende des Luftströmungskanals austritt, an dem die Strahlungsquelle angeordnet ist. Hierdurch sind die UV-Strahlung und der Luftstrom gleichgerichtet, so daß zur Hautbehandlung ein geführter Luftstrom in Verbindung mit der UV-Strahlung auf die Haut geleitet wird. In der zweiten Betriebsart ist es von Vorteil, wenn der vom Gebläse erzeugt Luftstrom aus dem Ende des Luftführungskanals austritt, an dem die Heizung angeordnet ist. Dieser Wechsel der Luftstromrichtung wird automatisch mit dem Wechsel der Betriebsart von dem Gebläse vorgenommen.

- 4 -

Vorteilhafte Weiterbildungen der Erfindung sind in den Unteransprüchen 3 bis 19 gekennzeichnet.

Die Zeichnungen veranschaulichen die Erfindung anhand eines Ausführungsbeispiels, und zwar zeigen:

Fig. 1 eine Seitenansicht der erfindungsgemäßen Vorrichtung in schematischer, teilweise geschnittener Darstellung,

Fig. 2 eine erfindungsgemäße Schaltung der Vorrichtung in Fig. 1,

Fig. 3,4 eine erfindungsgemäße Schaltung der Vorrichtung in alternativer Ausführung,

Fig. 5 eine weitere Ausführung einer erfindungsgemäßen Schaltung.

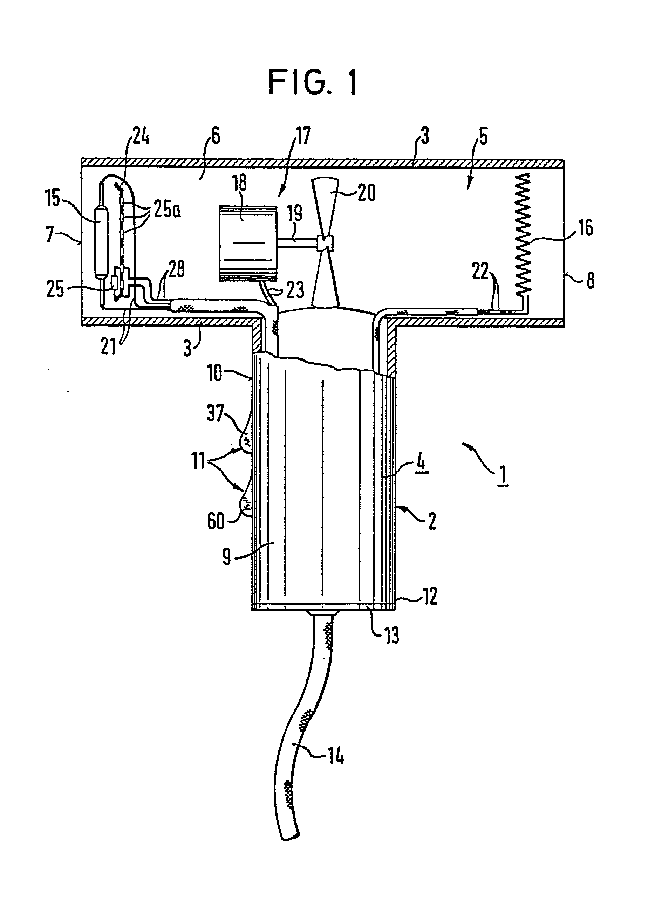

Die Figur 1 zeigt eine erfindungsgemäße Vorrichtung 1, die sowohl der Hautbestrahlung mit ultravioletter Strahlung als auch der Haartrocknung dient. Die Vorrichtung 1 weist ein T-förmiges Gehäuse 2 auf, das aus einem ersten Schenkel 3 besteht, an den etwa mittig zu seiner Längserstreckung ein zweiter Schenkel 4 angesetzt ist. Nach einer bevorzugten Ausführungsform der Erfindung sind der erste Schenkel 3 und der zweite Schenkel 4 im Querschnitt kreisförmig ausgebildet. Das Innere 5 des ersten Schenkels 3 bildet einen Luftführungskanal 6, der einendig eine erste Luftöffnung 7 und anderendig eine zweite Luftöffnung 8 besitzt. Der zweite Schenkel 4 des Gehäuses 2 ist als Handgriff 9

- 5 -

ausgebildet und weist an seiner Seitenwandung 10 Bedienelemente 11 auf. Das freie Ende 12 des Handgriffs 9 ist mit einer Bodenwandung 13 verschlossen, die von einer elektrischen Leitung 14 durchsetzt wird, welche der Zuführung von elektrischer Energie zu der erfindungsgemäßen Vorrichtung 1 dient.

Erfindungswesentlich ist, daß in dem Luftführungskanal 6 einendig, und zwar im Bereich der ersten Luftöffnung 7, eine ultraviolette Strahlung abgebende Strahlungsquelle 15 und anderendig, und zwar im Bereich der zweiten Luftöffnung 8, eine elektrische Heizung 16 (Heizwicklung) angeordnet ist. Zwischen der Strahlungsquelle 15 und der Heizung 16 befindet sich im Luftführungskanal 6 ein Gebläse 17, das einen Elektromotor 18 und ein mit der Welle 19 des Elektromotors 18 verbundenes Lüfterrad 20 besitzt. Von der Strahlungsquelle 15, der Heizung 16 und dem Gebläse 17 führen elektrische Leitungen 21, 22 und 23 in den Handgriff 9 hinein, wo sie mit einer in der Figur 1 nicht sichtbaren elektrischen Schaltung bzw. mit den Bedienelementen 11 oder gegebenenfalls mit der elektrischen Leitung 14 verbunden sind. Hinter der Strahlungsquelle 15, das heißt auf ihrer dem Gebläse 17 zugewandten Seite ist ein Reflektor 24 angeordnet, der von Luftdurchtrittsöffnungen 25a durchsetzt wird. In unmittelbarer Nähe der Strahlungsquelle 15 ist ein die Temperatur der Strahlungsquelle 15 erfassender NTC-Widerstand 25 angeordnet, der über Leitungen 26 mit der in dem Handgriff 9 untergebrachten elektrischen Schaltung verbunden ist.

- 6 -

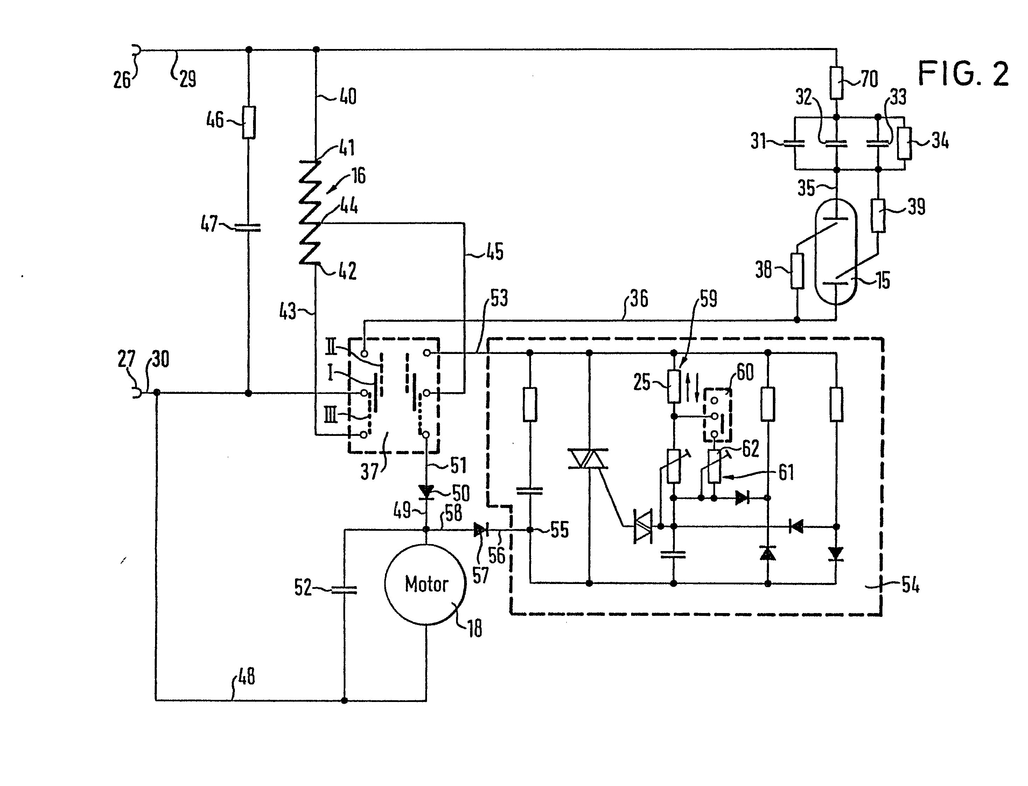

Die Figur 2 zeigt eine erfindungsgemäße Schaltung der Vorrichtung 1. Die Anschlußklemmen 26 und 27 der erfindungsgemäßen Schaltung werden an die Wechselspannung eines elektrischen Versorgungsnetzes angeschlossen, wobei die von den Anschlußklemmen 26 und 27 abgehenden Leitungen 29 und 30 der elektrischen Leitung 14 in Figur 1 entsprechen. Die Anschlußklemme 26 ist über die Leitung 29 einendig an einen Strombegrenzungswiderstand 30 angeschlossen, der anderendig mit einer Parallelschaltung aus drei Kondensatoren 31, 32 und 33 verbunden ist, zu denen ein Entladewiderstand 34 parallel geschaltet ist. Die parallel geschalteten Kondensatoren 31, 32 und 33 bilden einen kapazitiven Vorwiderstand für die Strahlungsquelle 15, deren eine Elektrode über eine Leitung 35 an diesen kapazitiven Vorwiderstand angeschlossen ist. Die andere Elektrode der Strahlungsquelle 15, die als Quecksilberdampf-Hochdrucklampe (UV-Lampe) ausgebildet ist, steht über die Leitung 36 mit einem Schalter 37 in Verbindung. Die beiden Elektroden der Strahlungsquelle 15 sind über Widerstände 38 bzw. 39 mit entsprechenden Zündelektroden der Strahlungsquelle 15 verbunden.

Die an die Anschlußklemme 26 angeschlossene Leitung 29 steht über eine Leitung 40 mit dem einen Ende 41 der als Widerstandsdrahtwicklung ausgebildeten Heizung 16 in Verbindung. Das andere Ende 42 der Heizung 16 ist über eine Leitung 43 mit dem Schalter 37 verbunden. Ein Wicklungsabgriff 44 steht über die Leitung 45 mit dem Schalter 37 in Verbin- dung. Parallel zu den Anschlußklemmen 26 und 27 ist eine Reihenschaltung aus einem Widerstand 46 und einem Kondensator 47 geschaltet, die als Entstörglied arbeitet. Die Anschlußklemme 27 steht über die Leitung 30 und eine Leitung 48 mit einem Pol des Elektromotors 18 in Verbindung, wobei der andere Pol des Elektromotors 18 über eine Leitung 49 an die Katode einer Diode 50 angeschlossen ist. Die Anode der Diode 50 ist über eine Leitung 51 mit dem Schalter 37 verbunden. Parallel zu dem Elektromotor 18 liegt ein Entstör- und Pufferkondensator 52. Eine Leitung 53 führt von dem Schalter 37 zu einer Phasenanschnittsschaltung 54, dessen Ausgangsanschluß 55 über eine Leitung 56 an die Katode einer Diode 57 angeschlossen ist. Die Anode der Diode 57 steht über eine Leitung 58 mit der Leitung 49 in Verbindung.

Die Phasenanschnittsschaltung 54 besitzt einen Diac und einen Triac und ist herkömmlicher Art und Weise aufgebaut, so daß auf diese hier nicht näher eingegangen zu werden braucht. Das phasenanschnittswinkelbestimmende Glied 59 der Phasenanschnittsschaltung 54 ist der NTC-Widerstand 25, der gemäß der obigen Beschreibung in unmittelbarer Nähe der Strahlungsquelle 15 zur Ermittlung deren Temperatur angeordnet ist. Weiterhin weist die Phasenanschnittsschaltung 54 einen Schalter 60 auf, bei dessen Betätigung ein weiteres phasenanschnittswinkelbestimmendes Element 61, das als Potentiometer 62 ausgebildet ist, ein- bzw. abgeschaltet werden kann.

Liegt an den Anschlußklemmen 26 und 27 Netzspannung

- 8 -

an, und befindet sich der Schalter 37 in seiner
mit durchgezogenen Linien dargestellten Stellung I,
so befindet sich die erfindungsgemäße Vorrichtung
außer Betrieb, das heißt die Schalterstellung I
entspricht der Stellung "Aus". Wird der Schalter
37, der einem Bedienelement 11 (Figur1) entspricht, in seine
gestrichelt dargestellte Stellung II verbracht,
so wird die Strahlungsquelle 15 über ihren kapazitiven Vorwiderstand an Netzspannung gelegt. Gleichzeitig verbindet der Schalter 37 in seiner Stellung
II die Phasenanschnittsschaltung 54 über einen als
Vorwiderstand dienende Teilabschnitt der Widerstandsdrahtwicklung der Heizung 16 mit der Netzspannung.
Der Teilabschnitt der Widerstandsdrahtwicklung
wird von dem Bereich gebildet, der zwischen dem
Ende 41 und dem Wicklungsabgriff 44 liegt. Der Ausgangsanschluß 55 der Phasenanschnittsschaltung 54
ist über die Diode 57 mit dem Elektromotor 18 verbunden, so daß dieser je nach eingestelltem Phasenanschnittswinkel mit einer bestimmten Drehzahl läuft.
Wird der Schalter 37 in seine punktiert dargestellte Stellung III gebracht, so werden die beiden Enden 41 und 42 der Heizung 16 an Netzspannung gelegt,
so daß sich die Heizung erwärmt. Ferner wird der
Elektromotor 18 über den gleichen Teilabschnitt
der Widerstandsdrahtwicklung · der Heizung 16 - wie
schon bei der Schalterstellung II beschrieben -
über die Diode 50 an Netzspannung gelegt. Da die
Diode 50 gegenüber der Diode 57 in umgekehrter
Durchlaßrichtung an den Elektromotor 18 angeschlossen ist, wird dieser gegenüber dem Betrieb bei der
Schalterstellung II eine umgekehrte Drehrichtung annehmen.

Befindet sich der Schalter 37 in seiner Schaltstellung II, das heißt der Motor wird über die Phasenanschnittsschaltung 54 betrieben, so kann durch Betätigung des Schalters 60, der eines der Bedienelemente 11 (Figur 1) darstellt, der Phasenanschnittswinkel der Phasenanschnittsschaltung 54 derart verändert werden, daß sich die Motordrehzahl des Elektromotors 18 um einen gewissen Betrag erhöht.

Das den Phasenanschnittswinkel bestimmende Glied 59, das als NTC-Widerstand 25 ausgebildet ist, befindet sich - wie oben schon beschrieben - in unmittelbarer Nähe der Strahlungsquelle 15, so daß die von der Strahlungsquelle ausgehende Wärme von dem NTC-Widerstand 25 registriert wird. Je nach Wärmebeaufschlagung des NTC-Widerstandes 25 verändert dieser einen Widerstandswert, wodurch der Phasenanschnittswinkel der Phasenanschnittsschaltung 54 verändert wird und damit Einfluß auf die Motordrehzahl des Elektromotors 18 genommen wird. Mit der Veränderung der Motordrehzahl ändert sich jedoch die von dem Gebläse 17 geförderte Luftmenge, die bei der erfindungsgemäßen Vorrichtung zur Kühlung der Strahlungsquelle 15 verwendet wird; denn die Strahlungsquelle 15 befindet sich in Luftführungskanal 6 und steht über die Luftdurchtrittsöffnungen 25a des Reflektors 24 mit dem Luftstrom in Verbindung. Durch Anordnung des NTC-Widerstandes 25 derart, daß er die Temperatur der Strahlungsquelle 15 erfaßt, stellt die Phasenanschnittsschaltung 54 ein Teilelement eines Regelkreises dar,

- 10 -

der dazu dient, die Temperatur der Strahlungsquelle 15 auf einen konstanten, vorgegebenen Wert zu
halten. Zur Beschreibung des Regelkreises wird davon ausgegangen, daß die Temperatur der
Strahlungsquelle 15 aus irgendeinem Grunde ansteigt.
Hierdurch steigt auch die Temperatur des in unmittelbarer Nachbarschaft liegenden NTC-Widerstandes
25 an, so daß sich sein Widerstandswert ebenfalls
verändert, wodurch der Phasenanschnittswinkel der
Phasenanschnittsschaltung 54 derart verändert wird,
daß dem Elektromotor 18 eine höhere Spannung zugeführt wird. Damit erhöht sich auch die Drehzahl
des Elektromotors 18, wodurch eine größere Luftmenge in dem Luftführungskanal 6 gefördert wird,
was wiederum zu einer intensiveren Kühlung der
Strahlungsquelle 15 führt, so daß sich die Temperatur an der Strahlungsquelle 15 wieder erniedrigt.
Sinkt die Temperatur aus irgendeinem Grunde unter
den gewünschten Wert, so wirkt die Regelschaltung
im entgegengesetzten, analogen Sinne, wie eben beschrieben. Durch Grundeinstellung der Phasenanschnittsschaltung 54 läßt sich somit die Temperatur
an der Strahlungsquelle 15 auf einem gewünschten
Wert halten. Dieses ist deshalb notwendig, weil
die UV-Strahlungsquelle 15 nur bei einer bestimmten Temperatur eine bestimmte gewünschte Strahlungsleistung und auch eine bestimmte Wellenlänge der
Strahlung abgibt, die für einen optimalen Behandlungserfolg bei der Bestrahlung von Hauterkrankungen notwendig sind. Ferner ermöglicht die geregelte Kühlung der UV-Strahlungsquelle einen Langzeitbetrieb, der bei Nichtkühlung der UV-Strahlungsquelle 15 nicht möglich wäre, weil

nach wenigen Minuten ein Erlöschen der Strahlungsquelle erfolgen würde. Der Grund für das Erlöschen der UV-Strahlungsquelle ist die Verwendung eines kapazitiven Vorwiderstandes. Mit steigender Betriebszeit erhöht sich nicht nur die Temperatur der UV-Strahlungsquelle 15 sondern auch hiermit ihre Wiederzündspannung. Bei jeder Zündung der Strahlungsquelle 15, die in jeder Halbwelle der Netzspannung erfolgt, treten Störspannungen (Rückwirkungen) auf, deren Frequenz höher als die der Netzspannung ist. Hierdurch erniedrigt sich der Blindwiderstand des kapazitiven und somit frequenzabhängigen Vorwiderstandes, so daß die Spannung an der Strahlungsquelle 15 ansteigt. Dieses führt jedoch zu einer Erhöhung des Strahlungsquellenstromes und gleichzeitig damit zur Temperaturerhöhung, was wiederum eine Erhöhung der Wiederzündspannung zur Folge hat. Gleichzeitig werden hiermit die Störfrequenzen verstärkt, was somit eine weitere Erniedrigung des Widerstandswertes des kapazitiven Vorwiderstandes bewirkt. Diese Vorgang schaukelt sich auf, bis die von der UV-Strahlungsquelle 15 benötigte Wiederzündspannung größer als die zur Verfügung stehende Netzspannung ist. Eine derartig hohe Spannung kann der UV-Strahlungsquelle 15 jedoch nicht zur Verfügung gestellt werden, so daß diese erlischt. Die mit der Phasenanschnittsschaltung 54 verwirklichte Regelschaltung ermöglicht jedoch durch Regelung des Luftstromes eine derartige Kühlung der Strahlungsquelle 15, daß der Momentanwert ihrer Wiederzündspannung $\le$ dem Momentanwert ihrer Versorgungsspannung ist. Hierdurch ist ein nichtgewünschtes Erlöschen der UV-Strahlungsquelle ausgeschlossen. Darüber hinaus kann der Arbeitspunkt der UV-Strahlungsquelle derart eingestellt werden, daß für optimale Behandlungserfolge eine gewünschte Strahlungsleistung und auch die gewünschte Wellenlänge der Strahlung abgegeben werden. Durch Betätigung des Schalters 60 kann die Luftstrommenge erhöht werden, wodurch eine gewünschte Arbeitspunktverschiebung stattfindet, die dazu führt, daß die Strahlungsquelle in ihrer Strahlungsleistung und der abgegebenen Wellenlänge verändert wird und darüber hinaus ein erhöhter Luftstrom auf die zu behandelnde Hauterkrankung abgegeben wird.

Anhand der Figur 1 soll nunmehr der Betrieb der erfindungsgemäßen Vorrichtung 1 erläutert werden. Wird der Schalter 37 in seine Schaltstellung II verbracht, was der ersten Betriebsart entspricht, bei der eine Hautbestrahlung vorgenommen werden kann, so wird die UV-Strahlungsquelle 15 eingeschaltet und von dem Gebläse 17 ein Luftstrom derart durch den Luftführungskanal 6 gefördert, daß dieser in die zweite Luftöffnung 8 eintritt und aus der ersten Luftöffnung 7 austritt. Zur Behandlung einer Hauterkrankung muß somit die erfindungsgemäße Vorrichtung mit auf die Behandlungsstelle gerichteter erster Luftöffnung 7 verwendet werden, wobei ein bestimmter Bestrahlungsabstand einzuhalten ist. Wird der Schalter 37 in seine Stellung III verbracht, so entspricht dieses der zweiten Betriebsart, bei der die erfindungsgemäße Vorrichtung als Haartrockner dient. In dieser Betriebsart ist die UV-Strahlungsquelle 15 aus- und die Heizung 16

- 13 -

eingeschaltet. Das Gebläse hat gegenüber der ersten Betriebsart seine Drehrichtung umgekehrt, so daß ein Luftstrom derart durch den Luftführungskanal 6 gefördert wird, daß dieser in die erste Luftöffnung 7 eintritt und aus der zweiten Luftöffnung 8 austritt. Eine Trocknung von nassen Haaren kann mit der erfindungsgemäßen Vorrichtung dadurch vorgenommen werden, daß die Vorrichtung mit den Haaren zugewandter zweiter Luftöffnung 8 in einem gewünschten Abstand gehalten wird, so daß der warme Luftstrom auf die Haare trifft.

Wie sich aus Fig. 2 ergibt, ist der Strahlungsquelle 15 ein kapazitiver Vorwiderstand 31, 32, 33 vorgeschaltet. Dieser kapazitive Vorwiderstand weist etwa eine Größe von 30 µF auf. Kondensatoren, die zur Erzeugung eines derartigen Widerstandswertes in Verbindung mit der anliegenden Versorgungsspannung für die Strahlungsquelle 15 geeignet sind, sind aber relativ groß und zudem muß eine Vielzahl von Kondensatoren parallel geschaltet werden, so daß sich ein großer Platzbedarf im Gehäuse und ein hohes Gewicht ergeben. Zudem sind derartige Kondensatoren relativ teuer, so daß sich durch deren Verwendung der Preis der erfindungsgemäßen Vorrichtung wesentlich erhöht.

Es liegt deshalb der Erfindung weiterhin die Aufgabe zugrunde, diese vorstehenden Nachteile zu vermeiden und eine Möglichkeit zu schaffen, bei gleicher Schaltungscharakteristik der Strahlungsquelle 15 wie in Fig. 2, eine Reduzierung des kapazitiven Vorwiderstandes zu erreichen. Dabei muß sichergestellt sein, daß stets die vorgeschriebene Betriebsspannung der Strahlungsquelle

- 14 -

anliegt, ihre Zündung gewährleistet ist und der Betriebsstrom begrenzt wird.

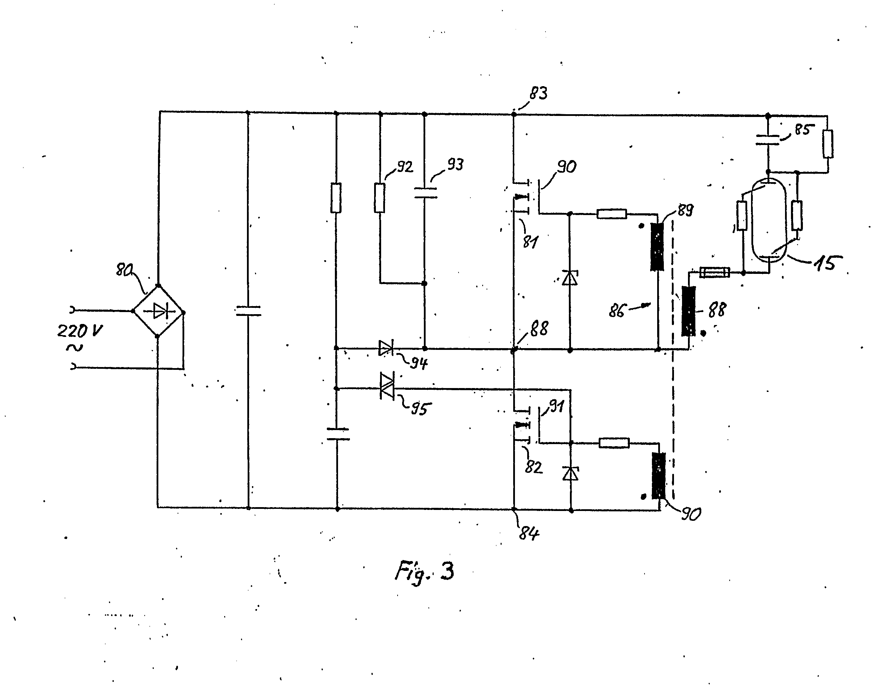

Erfindungsgemäß wird dies dadurch erreicht, daß, wie
in Fig. 3 gezeigt, ein Schaltnetzteil zur Erzeugung
der erforderlichen Versorgungsspannung der Strahlungsquelle 15 benutzt wird. Bei der erfindungsgemäßen
Schaltung ist die Strahlungsquelle 15 selbst ein Teil
eines selbstschwingenden Systems.

Gemäß Fig. 3 wird aus der Netzspannung von z.B. 220 Volt
Wechselspannung mit Hilfe eines Netzgleichrichters 80
von bekannter Bauart eine Gleichspannung von ca. 300 Volt
gewonnen. Mit Hilfe von zwei in Reihe liegenden, elektronischen Schaltern 81, 82 wird eine Rechteckspannung
erzeugt, deren Frequenz von der Zündung der die elektronischen Schalter bildenden Schalttransistoren abhängt. Bei den verwendeten Schalttransistoren handelt
es sich um Leistungs-Feldeffekttransistoren (VMOS-FET),
die in beiden Richtungen betrieben werden können. Die
in Reihe liegenden elektronischen Schalter 81, 82 sind
zwischen den Punkten 83, 84 angeordnet.

Ein Schwingkreis, bestehend aus einem Kondensator 85,
einem Leistungsübertrager 86 und einer weiteren kleiner dimensionierten Spule, die im Leistungsübertrager mit enthalten ist, bestimmt das Frequenzverhalten der erfindungsgemäßen Schaltung. Bei
dieser Schaltung bildet die Kapazität 85 den kapazitiven Vorwiderstand der Strahlungsquelle 15. Der verwendete Leistungsübertrager, dessen Leistung frequenzabhängig ist,
weist eine Hauptwicklung 87 auf, die mit der Strahlungsquelle 15 einendig verbunden ist und deren anderes Ende zwischen den beiden elektronischen Schaltern 81,
82 angeschlossen ist, und zwar am Punkt 88. Der

- 15 -

Leistungsübertrager weist zwei Nebenwicklungen 89, 90
auf, die gegensinnig gewickelt sind. Dabei ist die eine
Wicklung 89 einendig mit dem Gate 90 des elektronischen
Schalters 81 verbunden und anderendig mit dem Punkt 88,
und die andere Nebenwicklung 90 ist einendig mit dem
Gate 91 des elektronischen Schalters 82 verbunden und
anderendig mit dem Punkt 84. Der Punkt 88 bildet einen
virtuellen Massepunkt in der Schaltung. Die beiden elektronischen Schalter öffnen abwechselnd, und zwar in
Abhängigkeit von der Stromrichtung in der Hauptwicklung 87. Die erfindungsgemäße Schaltung schwingt bei
einer Frequenz von ca. 140 kHz an. Bei dieser Frequenz
kann die erfindungsgemäße Strahlungsquelle 15 zünden.
Durch die Belastung verändert sich die Kreisfrequenz auf
ca. 200 kHz. Durch die erfindungsgemäß vorgesehenen
hohen Frequenzen ist es möglich, kleine Bauteile mit gutem Leistungsverhalten zu koppeln. Der Schwingkreis wird durch einen
Hilfsoszillator 92a mit einer Impulsfrequenz von ca. 400-1000 kHz
in Bewegung gesetzt; dieser Hilfsoszillator besteht aus der
Parallelschaltung des Widerstandes 92 und des Kondensators 93 und
der Diode 94 sowie einem Diac 95. Mit Hilfe der Nebenspulen 89,90
wird das Gegentaktsignal zur weiteren Zündung der elektronischen Schalter erzeugt.

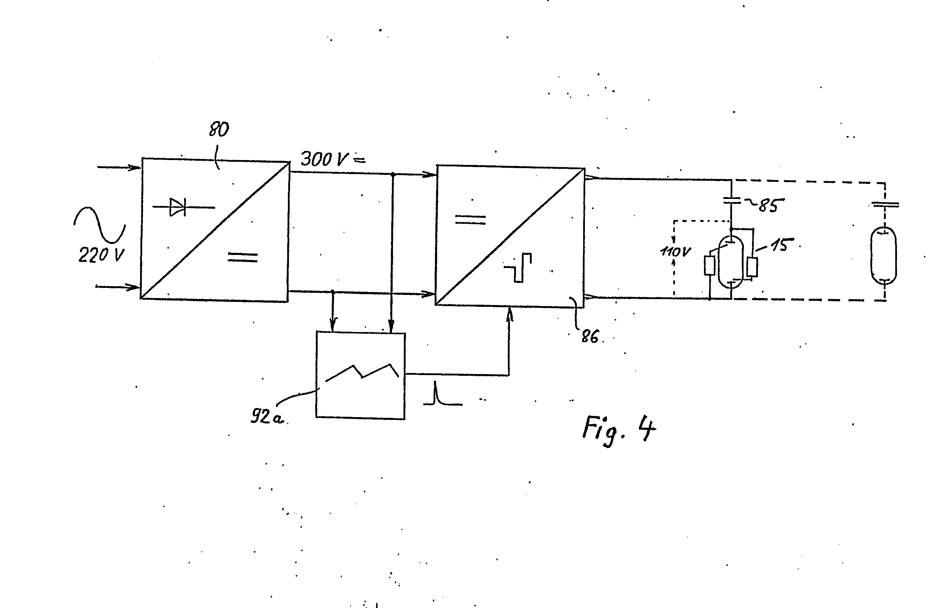

In Fig. 4 ist das Blockschaltbild der erfindungsgemäßen
Schaltung dargestellt.

Ein wesentlicher Vorteil der erfindungsgemäßen Schaltung
ist die völlige Trennung der Strahlungsquelle 15 vom
Netz, was erheblich zur Entstörung und Sicherheit beiträgt. Auch ist die Verwendung von zwei oder mehr Strahlern an einer erfindungsgemäßen Schaltung möglich. Auch
sehr hohe Zündströme stellen für die erfindungsgemäß
verwendeten Bauelemente keine großen Probleme dar, da die erfindungsgemäße Schaltung bei Spitzenbelastungen "in die Knie" geht und die Betriebsspannung heruntersetzt. Auch ist die Umsetzung auf andere Netz-, Spannungs- und Frequenzverhältnisse ohne weiteres möglich. Daneben ermöglicht die erfindungsgemäße Schaltung Eingriffe in das Leistungsverhalten der Schaltung, und zwar ohne wesentliche Änderungen.

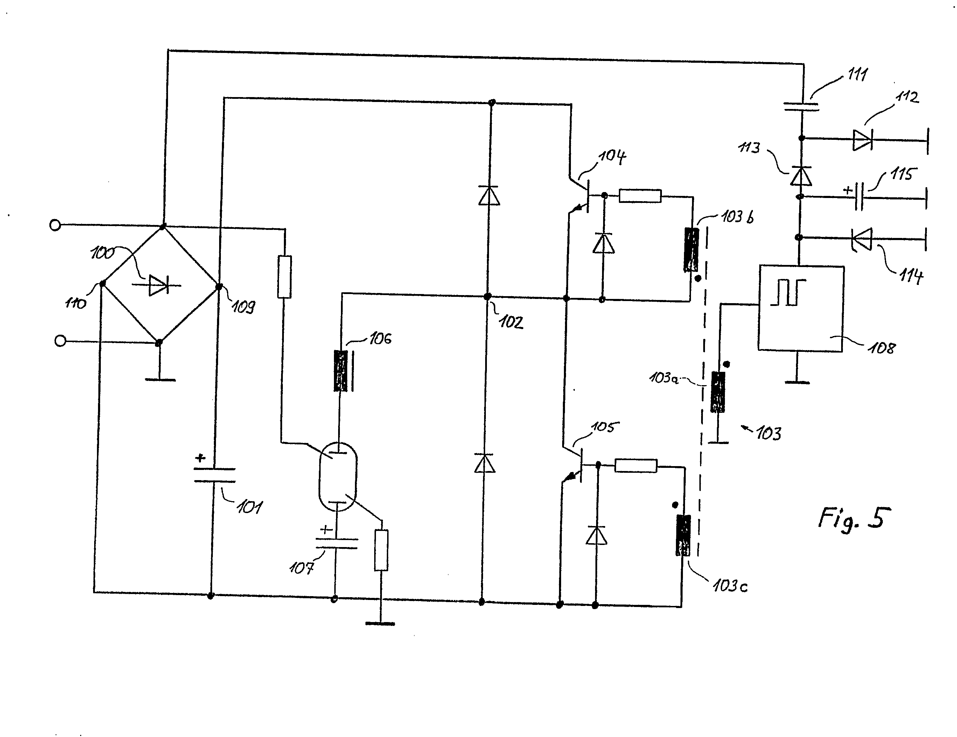

In Fig. 5 ist eine weitere erfindungsgemäße Schaltung zum Betreiben der Strahlungsquelle 15 dargestellt. Bei dieser wird die Netzspannung von z.B. 220 Volt Wechselspannung über einen Gleichrichter 100 und einen Kondensator 101 in eine gesiebte Gleichspannung von ca. 300 Volt gleichgerichtet. Diese gleichgerichtete Spannung wird nun unter Bezug auf eine virtuelle Masse 102 zerhackt. Dies erfolgt über ein Schaltnetzteil, bestehend aus einem frequenzabhängigen Leistungsübertrager 103 und zwei Schalttransistoren 104 und 105. Bei diesen Schalttransistoren handelt es sich um handelsübliche Transistoren mit einer Spannungsfestigkeit von ca. 400 Volt. Die zerhackte Gleichspannung wird über eine Reihenschaltung einer Spule 106 als Vorwiderstand und der Strahlugnsquelle 15 und einem Kondensator 107 an den virtuellen Massenpunkt gelegt. Durch den induktiven Widerstand der Spule 106 wird der Strom durch die Strahlungsquelle 115 begrenzt. Durch den Kondensator 107 wird der Strom durch die Strahlungsquelle gleichspannungsfrei gehalten. Der induktive Widerstand der Spule liegt bei ca. 60 Ohm. Bei einer Betriebsfrequenz zwischen 50 und 100 kHz ergibt sich die entsprechende Spulengröße.

Die Ansteuerung der beiden Schalttransistoren 104 und 105 erfolgt im Gegentakt mit Hilfe des Leistungsübertragers 103. Dieser Leistungsübertrager 103 besitzt eine Hauptspule 103a, die einendig an einen Spannungsgenerator 108 und anderendig an Masse liegt, und zwei gegensinnig gerichtete Nebenspulen 103b und 103c. Der Spannungsgenerator erzeugt eine alternierende Rechteckspannung mit einer Frequenz zwischen etwa 80 und 120 kHz. Aufgrund des gegensinnigen Wicklungssinns der Nebenspulen 103b und 103c werden die beiden Schalttransistoren 104 und 105 im Gegentakt geöffnet und geschlossen, so daß entsprechend die Strahlungsquelle 15 spannungsmäßig gesteuert wird, wobei die Betriebsspannung zwischen der virtuellen Masse 102 und jeweils abwechselnd den Punkten 109 und 110 anliegt. Der Impulsgenerator 108 wird über die Schaltung aus dem Kondensator 11, den Dioden 112, 113, 114 und dem Kondensator 115 aus der Netzspannung versorgt.

Die Schalttransistoren 104 und 105 werden, wie bereits ausgeführt, durch die Nebenspulen 103b und 103c angesteuert, die über übliche Vorwiderstände die Basis der Schalttransistoren ansteuern.

Die erfindungsgemäße Schaltung ermöglicht sehr geringe Bauteilkosten bei einer hohen Betriebssicherheit, da die Ansteuerung unabhängig von den Netzgrößen erfolgt. Die erfindungsgemäße Verwendung eines induktiven Vorwiderstandes für die Strahlungsquelle verhindert ein Auftreten von Spitzenströmen, und durch die erfindungsgemäße Schaltung können eine Vielzahl von Strahlungsquellen parallel betrieben werden, ohne daß die Schaltung prinzipiell geändert werden müßte.

M 259 EPA

## Patentansprüche

1. Vorrichtung zur Hautbestrahlung sowie Haartrocknung, mit einem Luftführungskanal, in dem ein Gebläse, eine Luftstromheizung und eine ultraviolette Strahlung abgebende Strahlungsquelle angeordnet ist, wobei in einer ersten Betriebsart die eingeschaltete Strahlungsquelle bei der Bestrahlungsbehandlung von einem vom Gebläse erzeugten Luftstrom gekühlt wird, und in einer zweiten Betriebsart ein von dem Gebläse erzeugter und von der Heizung angewärmter, austretender Luftstrom zur Haartrocknung dient, d a d u r c h   g e k e n n z e i c h n e t , daß sich in dem Luftführungskanal (6) einendig die Strahlungsquelle (15), anderendig die Heizung (16) und zwischen der Strahlungsquelle (15) und der Heizung (16) das Gebläse (17) befindet.

2. Vorrichtung nach Anspruch 1, d a d u r c h   g e k e n n z e i c h n e t , daß das Gebläse (17) mit einem Wechsel der Betriebsart die Richtung des Luftstroms in dem Luftführungskanal (6) umkehrt.

- 2 -

3. Vorrichtung nach Anspruch 1, oder 2, d a d u r c h g e - k e n n z e i c h n e t , daß der Luftführungs- kanal (6) zwei Luftöffnungen (7,8) aufweist, daß im Bereich der ersten Luftöffnung (7) die Strahlungsquelle (15) und im Bereich der zweiten Luftöffnung (8) die Heizung (16) angeordnet ist, daß in der ersten Betriebsart der vom Gebläse (17) erzeugte Luftstrom in die zweite Luftöffnung (8) ein- und aus der ersten Luftöffnung (7) austritt, und daß in der zweiten Betriebsart der vom Gebläse (17) erzeugte Luftstrom in die erste Luftöffnung (7) ein- und aus der zwei- ten Luftöffnung (8) austritt.

4. Vorrichtung nach einem der Ansprüche 1 bis 3, g e k e n n - z e i c h n e t d u r c h ein T-förmiges Ge- häuse (2), dessen erster Schenkel (3) den Luftführungskanal (6) bildet und dessen etwa in Längserstreckung des ersten Schenkels (3) mittig an diesen angesetzter zweiter Schenkel (4) als Handgriff (9) ausgebildet ist.

5. Vorrichtung nach einem oder mehreren der Ansprü- che 1 bis 4, d a d u r c h g e k e n n z e i c h- n e t , daß die Strahlungsquelle (15) über einen kapazitiven Vorwiderstand (31, 32,33) betrieben wird.

6. Vorrichtung nach einem oder mehreren der Ansprü- che 1 bis 5, d a d u r c h g e k e n n z e i c h- n e t , daß die Strahlungsquelle (15) im Luftstrom des Gebläses (17) angeordnet ist, und daß in der ersten Betriebsart der Luftstrom die Strah-

- 3 -

lungsquelle (15) derart kühlt, daß der Momentanwert ihrer Wiederzündspannung $\leqq$
dem Momentanwert ihrer Versorgungsspannung ist.

7. Vorrichtung nach einem oder mehreren der Ansprüche 1 bis 6, d a d u r c h   g e k e n n z e i c h -
n e t,  daß das Gebläse (17) einen Elektromotor
(18) aufweist, dessen Drehrichtung zur Umkehrung
der Richtung des Luftstromes beim Wechsel der
Betriebsart umkehrbár ist.

8. Vorrichtung nach Anspruch 7, d a d u r c h   g e-
k e n n z e i c h n e t,  daß der Elektromotor
(18) in der ersten Betriebsart an eine Phasenanschnittsschaltung (54) zum Regeln des vom Gebläse (17)
abgegebenen Luftstroms angeschlossen ist.

9. Vorrichtung nach Anspruch 8, d a d u r c h   g e-
k e n n z e i c h n e t,  daß das phasenanschnittswinkelbestimmende Glied (59) der Phasenanschnittsschaltung (54) ein NTC-Widerstand (25) ist, der
zur Temperaturermittlung der Strahlungsquelle
(15) in deren Nähe angeordnet ist.

10. Vorrichtung nach Anspruch 8 oder 9, d a d u r c h
g e k e n n z e i c h n e t,  daß der vom Gebläse (17)
abgegebene Luftstrom durch die Phasenanschnittsschaltung (54) derart geregelt wird, daß die
Strahlungsquelle (15) eine konstante, vorwählbare
Temperatur aufweist.

11. Vorrichtung nach einem oder mehreren der Ansprüche
1 bis 10, d a d u r c h   g e k e n n z e i c h n e t,
daß durch Betätigung eines Schalters (60) ein
weiteres phasenanschnittswinkelbestimmendes

- 4 -

Element (61) der Phasenanschnittsschaltung (54)
derart veränderbar ist, daß hierdurch von dem
Gebläse (17) ein stärkerer Luftstrom abgegeben
wird.

12. Vorrichtung nach einem oder mehreren der Ansprüche 1 bis 11, d a d u r c h   g e k e n n z e i c h-
n e t,   daß die Strahlungsquelle (15) eine Quecksilberdampf-Hochdrucklampe (UV-Lampe) ist.

13. Vorrichtung nach einem oder mehreren der Ansprüche 1 bis 12, d a d u r c h   g e k e n n z e i c h-
n e t,   daß hinter der Strahlungsquelle (15)
ein mit Luftdurchtrittsöffnungen (25a) versehener
Reflektor (24) angeordnet ist.

14. Vorrichtung zur Hautbestrahlung sowie Haartrocknung mit einem Luftführungskanal, in dem ein
Gebläse, eine Luftstromheizung und eine ultraviolette Strahlung abgebende Strahlungsquelle
angeordnet ist, wobei die eingeschaltete Strahlungsquelle bei der Bestrahlungsbehandlung von
einem vom Gebläse erzeugten Luftstrom gekühlt
wird und die Strahlungsquelle über einen kompletten Vorwiderstand betrieben wird, insbesondere
nach einem oder mehreren der Ansprüche 1 bis 13, d a -
d u r c h   g e k e n n z e i c h n e t, daß die Strahlungsquelle
(15) mit einem Schaltnetzteil, bestehend aus einem frequenzabhängigen Leistungsübertrager (86;103) und gegensinnig angesteuerten elektronischen Schaltern
(81,82;104,105), angesteuert wird.

15. Vorrichtung nach Anspruch 14, d a d u r c h g e -
k e n n z e i c h n e t , daß die Strahlungsquelle
(15) und ihr kapazitiver Vorwiderstand (85) mit dem
Leistungsübertrager (86) einen Schwingkreis bildet,
dessen Kreisfrequenz im Bereich zwischen 140 und
200 kHz liegt.

16. Vorrichtung nach Anspruch 15, d a d u r c h g e -
k e n n z e i c h n e t , daß das Schaltnetzteil aus
der Wechselstromnetzspannung eine Rechteckspannung
erzeugt, deren Frequenz von der Zündung der die elektronischen Schalter bildenden Schalttransistoren
(VMOS-FET) abhängt, deren Gate jeweils über gegensinnig gerichtete Nebenspulen (89,90) des Leistungsübertragers (86) angesteuert wird.

17. Vorrichtung nach Anspruch 14, d a d u r c h g e -
k e n n z e i c h n e t , daß als Vorwiderstand der
Strahlungsquelle eine Spule (106) dient und das
Schaltnetzteil (103) über einen von der Netzspannung
versorgten Impulsgenerator (108) angesteuert wird.

18. Vorrichtung nach Anspruch 17, d a d u r c h g e -
k e n n z e i c h n e t , daß der Impulsgenerator (108)
eine Impulsfrequenz von ca. 80 bis 120 kHz aufweist.

19. Vorrichtung nach Anspruch 17 oder 18, d a d u r c h
g e k e n n z e i c h n e t , daß das Schaltnetzteil
zwei gegensinnig gewickelte Nebenspulen (103b,103c)
aufweist, die jeweils die Basis eines Schalttransistors
(104,105) gegensinnig ansteuern und die Hauptspule
(103a) mit dem Impulsgenerator (108) verbunden ist.

FIG. 1

FIG. 2

Fig. 3

220 V
~

80
83
92
93
90
81
89
15
85
86
88
94
88
95
91
82
90
84

0158025

Fig. 4

80

300 V =

~85

110V

15

86

92a

220 V

Fig. 5

0158025

**Europäisches Patentamt**

**EUROPÄISCHER RECHERCHENBERICHT**

0158025

Nummer der Anmeldung

EP 85 10 1231

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-C- 939 646 (GÜNTHER) <br> * Seite 2, Zeilen 21-61 * | 1,6 | A 61 N 5/06 |
| A | FR-A-2 471 158 (BOSCH) <br> * Seite 1, Zeile 29 - Seite 2, Zeile 6 * | 1 | |
| A | FR-A-2 447 161 (L'OREAL) <br> * Seite 8, Zeilen 21-28 * | 1 | |
| D,A | DE-A-3 322 071 (KERSCHGENS) <br><br> * Zusammenfassung * | | |
| A | DE-A-2 832 143 (HIRSCH) <br> * Figur 1 * | 5 | RECHERCHIERTE SACHGEBIETE (Int Cl 4) |
| A | DE-A-1 464 661 (BAUMANN) <br> * Seite 2, Zeilen 12-16 * | 12,13 | A 61 N 5/00 <br> H 05 B 41/00 <br> A 45 D 20/00 |

Der vorliegende Recherchenbericht wurde für alle Patentansprüche erstellt.

| Recherchenort <br> DEN HAAG | Abschlußdatum der Recherche <br> 21-05-1985 | Prüfer <br> BERTIN M.H.J. |
|---|---|---|